# EUROPEAN PATENT APPLICATION

(11) **EP 2 878 597 A1**
(43) Date of publication of application: **03.06.2015**
(21) Application number: 13075078.9
(22) Date of filing: 28.11.2013
(51) Int. Cl.: C07D 301/12

(54) **Process for removal of 1,2-epoxy-5-hexene from epichlorohydrin**

(71) Applicant: Momentive Specialty Chemicals Research Belgium S.A., 1348 Ottignies Louvain-la-Neuve (BE)
(72) Inventor: Lienke, Joachim, 1348 Ottignies Louvain-La-Neuve (BE); van Rijn, Jimmy Antonius, 1348 Ottignies Louvain-La-Neuve (BE)

(57) **Abstract**

This invention concerns a process to remove 1,2-epoxy-5-hexene from epichlorohydrin which is produced by oxidation of unpurified allyl chloride comprising at least 1,5-hexadiene as impurity.

## Description

### Technical field

This invention concerns a process to remove 1,2-epoxy-5-hexene from epichlorohydrin which is produced by oxidation of unpurified allyl chloride comprising at least 1,5-hexadiene as impurity.

### Background art

The technical grade of allyl chloride, used in the production of epichlorohydrin (ECH), contains an impurity: 1,5-hexadiene. When ECH is made by epoxidation of such allyl chloride, part of this impurity is converted in 1,2-epoxy-5-hexene. Unfortunately, 1,2-epoxy-5-hexene has a boiling point of 118 to 121°C and hence cannot be separated by rectification from ECH (boiling temperature of 118°C).

The problem of 1,2-epoxy-5-hexene is augmented in processes where unreacted allyl chloride, and its impurity, are recycled in the process. In those processes the majority of 1,5 hexadiene is converted to 1,2-epoxy-5-hexene.

The US 4127594 described an efficient way to purify ECH by selective hydrogenation of the olefinic impurities. The 5,6-epoxyhexene-1 present in minor amount in ECH is selectively removed, with minimal concomitant destruction of ECH, by hydrogenation in the presence of a catalyst comprising rhodium, platinum or palladium deposited on a non-acidic, refractory support.

More recently, the CN 102417490 provides a process for purification of ECH containing 1,2-epoxy-5-hexene. In this reference the contaminant 1,2-epoxy-5-hexene is treated with excess chlorine or bromine at a temperature in the range of 5 to 30°C. The 1,2-epoxy-5-hexene was then converted into a product with a significantly higher boiling temperature. Next, remaining halogen was removed by flushing with nitrogen. Subsequently, rectification was used to obtain ECH with a purity of more than 99.8%.

The process of CN'490 is an elegant process. On the other hand, it has some significant disadvantages. It reduces the overall yield of the process due to the fact that the intermediate formed during the chlorination 1,2-epoxy-5-hexene can attack ECH yielding an addition product.

Furthermore, the reaction is carried out in a separate reactor, where the halogen is added to the ECH. Therefore, an extra reactor setup is required, increasing capital costs. Also chlorinated organic compounds are produced, which are generally harmful for the environment and do not have direct application. The reaction is carried out at temperatures that require active cooling of the ECH, even below ambient temperatures. The reaction temperature according to this reference is 0-30°C, preferably 0-15°C, more preferably 0-5°C.

Also, this reference specifically teaches that an excess of halogen has to be used (ratio halogen/1,2-epoxy-5-hexene between 1/1 and 3/1).

Moreover, after the reaction, the excess halogen must be removed (here by passing through nitrogen gas). This nitrogen stream needs to be treated to remove the halogen before being released into the atmosphere. Thus, there is a potential pollution issue with this process. This would again add complexity and costs.

The current inventors set out to solve the problem arising from allyl chloride containing 1,5-hexadiene impurity by an alternative process that is more economical, requires less investment and that might be used to create valuable side-products rather than waste.

### Disclosure of the invention

Accordingly, the current invention provides a process for removing 1,2-epoxy-5-hexene and 1,5 hexanediene from ECH by:
a) catalytic oxidation of a unpurified ECH mixture with 25-45 mole% of said 1,2-epoxy-5-hexene and 1,5 hexanediene whichare converted into 1,2,5,6-diepoxyhexane and
b) removing the light boiling components from a) and
c) further removing the 1,2,5,6-diepoxyhexane by a rectification step.

More specifically, it provides a process for removing 1,2-epoxy-5-hexene from ECH, which is produced by oxidation of unpurified allyl chloride comprising at least 1,5-hexadiene as impurity in more than 0.05 % by weight of 1,5-hexadiene, by:
(a) contacting a mixture comprising at least allyl chloride, ECH, 1,5-hexadiene, 1,2-epoxy-5-hexene and 1,2,5,6-diepoxyhexane, with an oxidant and a transition metal complex containing compound as catalyst.
(b) separating unreacted allyl chloride, unreacted 1,5-hexadiene and other low boiling components by distillation
(c) separating 1,2,5,6-diepoxyhexane and possibly other high boiling components from ECH and 1,2-epoxy-5-hexene by distillation whereby minimal 45% of the 1,5 hexadiene impurity is collected as 1,2,5,6-diepoxyhexane

The current invention allows to employ allyl chloride feedstocks in an epoxidation process that are contaminated with 1,5-hexadiene. The product of the contaminant is 1,2,5,6-diepoxyhexane, also known as 1,5-hexadiene diepoxide. This has a boiling point of 188°C (62°C at a reduced pressure of 300 mm Hg). Its boiling point is therefore significantly different from ECH, which facilitates the separation of the product. The 1,2,5,6-diepoxyhexane may be used, for instance in resin compositions. The latter is a significant advantage over other processes, like chlorination, where the products formed are considered useless.

### Mode for carrying out the invention

Step (a) is preferably carried out by contacting the organic phase of a mixture comprising at least allyl chloride, ECH, 1,5-hexadiene, 1,2-epoxy-5-hexene and 1,2,5,6-diepoxyhexane with an aqueous reaction medium wherein a homogenous catalyst is used, preferably a manganese complex or a tungsten containing polyoxometallate and a oxidant.

The preferred oxidant for the reaction is hydrogen peroxide. Other oxidants may be used, i.e. as precursor to the hydrogen peroxide, but given the availability and to reduce environmental impact hydrogen peroxide is the preferred oxidant. Hydrogen peroxide has strong oxidizing properties. It is typically used in an aqueous solution.

Preferably the oxidation is conducted in an aqueous reaction medium wherein a water-soluble transition metal complex is used as oxidation catalyst.

The catalyst preferably comprises a mononuclear manganese complex of the general formula (I) : [LMnX₃]Y (I) or a binuclear manganese complex of the general formula (II) : [LMn (µ-X)₃MnL]Y₂ (II) wherein Mn is a manganese; L or each L independently is a polydentate ligand, preferably a cyclic or acyclic compound containing 3 nitrogen atoms; each X independently is a coordinating species and each µ-X independently is a bridging coordinating species, selected from the group consisting of: RO⁻, Cl⁻, Br⁻, I⁻, F⁻, NCS⁻, N₃⁻, I₃⁻, NH₃, NR₃, RCOO-, RS03-, RS04-, OH⁻, O²⁻, O₂²⁻, HOO⁻, H₂O, SH⁻, CN⁻, OCN⁻, and S₄²⁻ and combinations thereof, wherein R is a C₁-C₂₀ radical selected from the group consisting of alkyl, cycloalkyl, aryl, benzyl and combinations thereof, and Y is an oxidatively-stable counterion.

Counterion Y may for instance be an anion selected from the group consisting of RO⁻, Cl⁻, Br⁻, I⁻, F⁻, SO₄²⁻, RCOO⁻, PF₆⁻, acetate, tosylate, triflate (CF₃SO₃⁻) and a combination thereof with R once again being a C₁ to C₂₀ radical selected from the group consisting of alkyl, cycloalkyl, aryl, benzyl and combination thereof. The type of anion is not very critical, although some anions are more preferred than others. A preferred counterion is PF₆⁻. Ligands which are suitable for the present invention are acyclic compounds containing at least 7 atoms in the backbone or cyclic compounds containing at least 9 atoms in the ring, each having the nitrogen atoms separated by at least two carbon atoms. A preferred class of ligands is that based on (substituted) triazacyclononane ("Tacn") . The prefer ligand is 1,4,7- trimethyl-1, 4 , 7 , -triazacyclononane ("TmTacn"), which is commercially available from for instance Aldrich. In this respect it is important to note that the water-solubility of the manganese catalyst is a function of all the aforementioned catalyst components. For instance, a mononuclear manganese complex prepared from MnS04 and TmTacn was found to be insufficiently soluble.

Dinuclear manganese complexes are preferred, because of their greater activity and solubility in water. Preferred dinuclear manganese complexes are those of the formula [Mn^{IV}₂ ([µ-0)₃L₂]Y₂, wherein L and Y have the meaning identified above, preferably TmTacn as ligand, and PF₆⁻ as counterion.

To achieve the high selectivity and turnover numbers of the current invention, the crude ECH mixture and oxidant are preferably reacted at a molar ratio of from 1:0.1 to 1:10, more preferably of from 1:0.2 to 1:1.2, still more preferably of from 1:0.8 to 1:1.

To ensure optimal oxidant efficiency, the oxidant is preferably added to the aqueous reaction medium at a rate about equal to the reaction rate of the catalytic oxidation. The catalytic oxidation may be performed in a batch process, in a continuous process or in a semi-continuous process. Indeed, the process may be modified in various aspects without departing from the gist of the invention.

Preferably, step (a) is performed in a common stirred tank reactor provided with a means of stirring. The catalyst, aqueous reaction medium and reactants may be added in batch, or the reactants may be added over a period of time. If hydrogen peroxide is added during the reaction, then it is added to either the (stirred) organic phase comprising the crude ECH or the (stirred) aqueous reaction medium. In (semi) continuous operations, various recycling streams may be used to control the reaction conditions and to optimize the production rate.

In terms of process design, a settler may be added to optimize the gravitational separation of the organic phase containing the ECH. Likewise, a membrane unit may be used to recycle the aqueous reaction medium with reduced loss of catalyst.

The reaction is conducted at or above atmospheric pressure. The precise pressure is not critical so long as the reaction mixture is maintained substantially in a non-gaseous phase. Typical pressures vary from about 1 to about 100 atmospheres. After step (a), the product comprising crude ECH which is subjected to rectification steps (b) and (c). Rectification conditions, such as distillation and fractional distillation, are known in the art.

Preferably, unreacted allyl chloride (for recycle purposes) and light ends, such as 1,5-hexadiene, chloropropanes and chloropropenes and the like, are removed as part of step (b) in one or more stages from the crude ECH first. The crude ECH is subjected to distillation, preferably in a perforated-plate column, bubble-cap plate column and/or packed column. This may be a single column or a series of columns.

The column is preferably equipped with an evaporation or heating device (or an evaporation or heating area or zone) located at or near the bottom of the column (or below the first plate). It is furnished with means to introduce an inlet stream at a point intermediate between the bottom and the top of the column; means to withdraw a lower-boiling stream at or near the top of the column, and means to withdraw a higher-boiling stream at or near the bottom of the column and possibly means to withdraw a product stream at an intermediate point on the column.

Preferably, a lower-boiling stream is continuously drawn off at the head of the column and a higher-boiling stream is continuously drawn off at the foot of the column.

Next, 1,2,5,6-diepoxy-hexane and other heavy end e.g., components such as monochlorohydrins and dichlorohydrins and the like, with boiling points greater than 118°C) are separated from ECH. This is again done in a distillation column or series of columns, operating however at lower pressure and/or higher temperature conditions then the conditions for removal of the light ends. Moreover, the product stream is drawn off at the head of the column or at an intermediate point between feed point and the head of the column.

According to the invention, the 1,2-epoxy-5-hexene in the ECH is converted into a higher boiling product, this is achieved by epoxidizing the mono-epoxide into the corresponding diepoxide. By ensuring that the 1,2-epoxy-5-hexene is converted into a high boiling product, this contaminant can effectively be removed. Moreover, this may lead to the production of 1,2,5,6-diepoxyhexane that can be cleanly separated and form an alternative attractive epoxy resin-type product.

In the preferred embodiment the reaction conditions during epoxidation of crude ECH are such that on molar basis at least 45% of the contaminant 1,5-hexadiene is converted into 1,2,5,6-diepoxyhexane and collected during distillation in the fraction that has a higher boiling point than ECH.

Desired reaction conditions according to this invention are those in which the rate of the consecutive reactions are such that formation of 1,2,5,6-diepoxyhexane from 1,2-epoxy-5-hexene is at least 0.45 that of the formation of 1,2-epoxy-5-hexene from 1,5 hexadiene. Somebody ordinary skilled in the art can derive those conditions from theoretical considerations as for example explained in Chemical Reactor Development, Dirk Thoenes or by variation of reactor types and reaction conditions such as phase ratio or residence time.

The most optimal reaction conditions for maximal removal of 1,2-epoxy-5-hexene can lead to reduced peroxide yields defined as the molar ratio of ECH produced over hydrogen peroxide charged. It can also lead to a reduction in turn over numbers defined as molar ratio of ECH produced over catalyst charged. Without being bound to any specific theory it is fair to assume that a high conversion of 1,2-epoxy-5-hexene to 1,2,5,6-diepoxyhexane is coupled to relatively low concentrations of allyl chloride in the reaction media which results in side reactions of the epoxidation becoming more favourable such as decomposition of peroxide and decomposition of ECH. Accordingly, this invention might be best practised when the removal of 1,2-epoxy-5-hexene is high, while the decrease in Yield and TON is small to nihil.

The present invention of removal of 1,2-epoxy-5-hexene via epoxidation can be combined with other techniques known in the art. This includes "bleeding" a fraction of the allyl chloride recycle stream removing low boiling side products of the allyl chloride productions that will accumulate or chlorination of remaining 1,2-epoxy-5-hexene in epichlorohydrin.

While the present invention has been described and illustrated by reference to particular embodiments, those of ordinary skill in the art will appreciate that the invention lends itself to variations not necessarily illustrated herein.

The following examples will more fully illustrate selected embodiments of this invention. All parts, percentages and proportions referred to herein and in the appended claims are by weight unless otherwise indicated.

### EXAMPLES

### Example 1:

In a typical continuous reaction, the flows of the components are 0.24 mol per hour of hydrogen peroxide, 9.0 micromol per hour of catalyst, 2.5 millimol per hour of oxalic acid and 0.79 mol per hour of crude ECH. pH is adjusted with sodium hydroxide and maintained between pH 3.2 and 4.0. Temperature is set at 15 °C and the reation volume is controlled at about 200 ml. The catalyst used is a [Mn₂(µ-O)₃{1,4,7-trimethyl-1,4,7-triazacyclononane}₂]²⁺ salt.

The starting allyl chloride contains < 0.4 wt% 1,5-hexadiene was used to produce the crude ECH. This results in a steady state yield based on the peroxide added of 72 % , a turnover number of 18500 and a diepoxide/monoepoxide (DO/BO) ratio of 1.

### Example 2:

As in example 1, but the allyl chloride contains 1.2 wt% 1,5-hexadiene as starting feedstock to produce the crude ECH. This results in a steady state yield based on the peroxide added of 69 %, a turnover number of 18500 and a DO/BO ratio of 1.

### Example 3

The product of the organic phase of example 1 and 2 are further purified by distillation according to a method as given above in the description.

## Claims

1. A process for removing 1,2-epoxy-5-hexene and 1,5 hexanediene from epichlorohydrin by:
a) catalytic oxidation of unpurified epichlorohydrin mixture with at least 45% of the moles of 1,5 hexanediene entering in the reactor are converted into 1,2,5,6-diepoxyhexane and
b) removing the light boiling components from a) and
c) further removing the 1,2,5,6-diepoxyhexane by a rectification step.

2. The process of claim 1, **characterized in that** the catalytic oxidation comprising a homogenous catalyst is used, preferably containing manganese or tungsten.

3. The process of claims 1 and 2, **characterized in that** the oxidant oxidizes the 1,5-hexadiene into 1,2-epoxy-5-hexene and the 1,2-epoxy-5-hexene into 1,2,5,6-diepoxyhexane, whereby the ratio 1,2-epoxy-5-hexene to 1,2,5,6-diepoxyhexane is smaller than 1 and the content of 1,2-epoxy-5-hexene within the isolated epichlorohydrin is less than 1.0 % by weight.

4. The process of claims 1 to 3, wherein the catalytic oxidation reaction is carried out in the presence of least an aqueous medium.

5. The process of claims 1 to 4, wherein the process is a continuous process.

6. The process of claim 2, **characterized in that** the catalytic oxidation comprising a mononuclear manganese complex of the general formula (I) : [LMnX₃]Y (I) or a binuclear manganese complex of the general formula (II) : [LMn (µ-X)₃MnL]Y₂ (II) wherein Mn is a manganese; L or each L independently is a polydentate ligand, preferably a cyclic or acyclic compound containing 3 nitrogen atoms; each X independently is a coordinating species and each µ-X independently is a bridging coordinating species, selected from the group consisting of: RO⁻, Cl⁻, Br⁻, I⁻, F⁻, NCS⁻, N₃⁻, I₃⁻, NH₃, NR₃, RCOO-, RS03-, RS04-, OH⁻, O²⁻, O₂²⁻, HOO⁻, H₂O, SH⁻, CN⁻, OCN⁻, and S₄²⁻ and combinations thereof, wherein R is a C₁-C₂₀ radical selected from the group consisting of alkyl, cycloalkyl, aryl, benzyl and combinations thereof, and Y is an oxidatively-stable counterion.

7. The process of claims 1 to 5, wherein the oxidant is hydrogen peroxide.

8. The process of claims 1 to 5, wherein the rectification step is done by a fractional distillation.

9. Use of the purified 1,2,5,6-diepoxyhexane according to claim 8 in resin compositions.
